(19) Europäisches Patentamt
European Patent Office
Office européen des brevets

(11) Numéro de publication: **0 161 235**
**A2**

(12) # DEMANDE DE BREVET EUROPEEN

(21) Numéro de dépôt: **85870061.0**

(22) Date de dépôt: **02.05.85**

(51) Int. Cl.[4]: **C 07 D 495/04**
A 61 K 31/435, C 07 D 333/36
//(C07D495/04, 333:00, 221:00)

(30) Priorité: **04.05.84 FR 8406970**

(43) Date de publication de la demande:
**13.11.85 Bulletin 85/46**

(84) Etats contractants désignés:
**AT BE CH DE FR GB IT LI LU NL SE**

(71) Demandeur: **SANOFI**
**40, Avenue George V**
**F-75008 Paris(FR)**

(72) Inventeur: **Giral, Louis Avenue du Père Soulas 1625**
**Villa No. 2 Les-Deux-Ruisseaux**
**F-34100 Montpellier(FR)**

(72) Inventeur: **Bompart, Jacques**
**55, La Cadoule**
**F-34160 Castries(FR)**

(72) Inventeur: **Puygrenier, Marc**
**26, avenue Jean Grandel**
**F-94360 Bry-sur-Marnes(FR)**

(74) Mandataire: **Cauchie, Daniel**
**c/o S.A. LABAZ-SANOFI N.V. Avenue De Béjar, 1**
**B-1120 Bruxelles(BE)**

(54) **Nouveaux dérivés de thiénopyridinone, leur procédé de préparation et compositions pharmaceutiques les contenant.**

(57) L'invention se rapporte à de nouveaux dérivés de thiénopyridinone de formule générale :

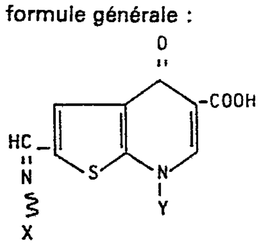

ainsi qu'à leurs sels pharmaceutiquement acceptables, dans laquelle Y représente l'hydrogène ou on groupement alkyle inférieur et X représent :

- un groupement hydroxy, alkanoyloxy inférieur, alkoxy inféreur non substitué ou substitué par un groupement hydroxy, alkoxy inférieur, di-(alkyle inférieur) amino, pipéridino, pyrrolidino, morpholino ou N-(alkyle inférieur) pipérazine,
- un groupement de formule :

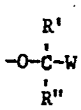 , 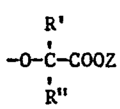 , 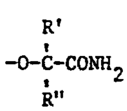 ou 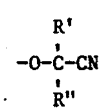

dans laquelle R' et R", qui sont identiques ou différents, représentent chacun l'hydrogène ou un radical alkyle inférieur, W représente l'hydrogène, un groupement alkyle inférieur ou un groupement vinyle ou éthynyle et Z représente l'hydrogène ou un radical alkyle inférieur,
- un radical phényle non substitué ou substitué par un atome de fluor, de chlore, de brome ou d'iode ou par un groupement hydroxy, alkoxy inférieur ou nitro,
- un groupement de formule :

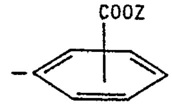

dans laquelle Z a la même signification que précédemment,
- un groupement amino, (alkyle inférieur) amino ou NHCOOZ dans lequel Z a la même signification que précédemment.

Les dérivés de thiénopyridinone de l'invention possèdent des propriétés antibactériennes.

EP 0 161 235 A2

Croydon Printing Company Ltd

NOUVEAUX DERIVES DE THIENOPYRIDINONE, LEUR PROCEDE DE PREPARATION ET LES COMPOSITIONS PHARMACEUTIQUES LES CONTENANT.

La présente invention se rapporte, d'une manière générale, à de nouveaux dérivés de thiénopyridinone doués d'activité antibactérienne, à leur procédé de préparation ainsi qu'à des compositions pharmaceutiques les contenant en tant que principes actifs.

En particulier, l'invention concerne les dérivés de thiénopyridinone de formule générale :

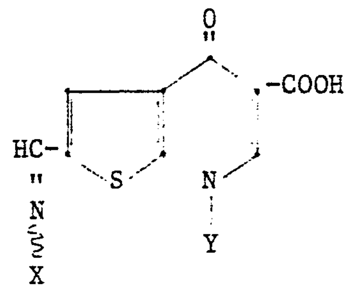

I

dans laquelle Y représente l'hydrogène ou un groupement alkyle inférieur et X représente :

- un groupement hydroxy, alkanoyloxy inférieur, alkoxy inférieur non substitué ou substitué par un groupement hydroxy, alkoxy inférieur, di-(alkyle inférieur)amino, pipéridino, pyrrolidino, morpholino ou N-(alkyle inférieur) pipérazino,
- un groupement de formule :

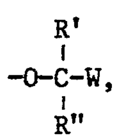 , 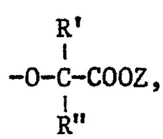 , 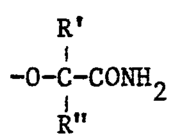 ou 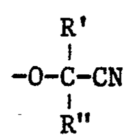

dans laquelle R' et R", qui sont identiques ou différents, représentent chacun l'hydrogène ou un radical alkyle inférieur, W représente l'hydrogène, un groupement alkyle inférieur ou un groupement vinyle ou éthynyle et Z représente l'hydrogène ou un radical alkyle inférieur,
- un radical phényle non substitué ou substitué par un atome de fluor, de chlore, de brome ou d'iode ou par un groupement hydroxy, alkoxy inférieur ou nitro,
- un groupement de formule :

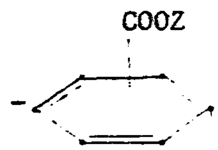

dans laquelle Z a la même signification que précédemment,

- un groupement amino, (alkyle inférieur)amino ou -NHCOOZ dans lequel Z a la même signification que précédemment.

Le terme "alkyle inférieur", tel qu'utilisé dans le présent contexte, désigne les restes d'hydrocarbure aliphatiques saturés contenant jusqu'à 4 atomes de carbone.

Les termes "alkanoyloxy inférieur" et "alkoxy inférieur" désignent, respectivement, les groupes carbonyloxy et hydroxyle substitués par des groupements alkyles inférieurs tels que définis ci-dessus.

En raison du groupement -CH=N∿∿-X, les composés de formule I peuvent exister sous forme d'isomères syn ou anti. Ces isomères pris individuellement et leurs mélanges sont également compris dans la présente invention.

De même, l'invention se rapporte aux sels pharmaceutiquement acceptables des composés de formule I.

Par sels pharmaceutiquement acceptables, on entend plus particulièrement les sels de métaux alcalins, tels que lithium, sodium ou potassium, les sels de métaux alcalino-terreux, tels que calcium ou magnésium ou les sels d'ammonium, tels que ceux obtenus à partir de l'ammoniac ou d'une amine, telle que la méthylamine, l'éthylamine, la diméthylamine, la diéthylamine, la triéthylamine, l'éthanolamine, la diéthanolamine, le trométamol et similaires.

Les composés de l'invention se sont révélés doués d'intéressantes propriétés antibactériennes susceptibles de les rendre utiles dans le traitement d'affections provoquées par le développement de bactéries pathogènes.

Selon un autre de ses aspects, la présente invention a pour objet un procédé pour la préparation des composés de formule I ci-dessus dans laquelle X a la même signification que précédemment ainsi que de leurs sels pharmaceutiquement acceptables, caractérisé en ce que l'on hydrolyse un ester de l'acide thiénopyridinonecarboxylique de formule générale :

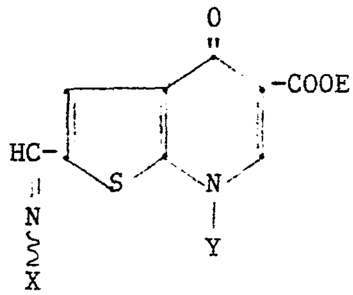

II

dans laquelle X et Y sont tels que définis ci-dessus et E représente un radical alkyle inférieur et, lorsque X est hydroxy, on soumet, si nécessaire, le produit ainsi obtenu à une réaction d'acylation avec un halogénure d'alkanoyle inférieur puis l'on transforme, si on le désire, le produit d'hydrolyse et d'acylation éventuelle en ses sels pharmaceutiquement acceptables.

L'hydrolyse peut être conduite selon les techniques de saponification connues par traitement à reflux avec une base minérale, telle que l'hydroxyde de sodium ou de potassium, suivi par une acidification avec un acide minéral, tel que l'acide chlorhydrique.

Lorsque, dans le composé de départ II, le substituant X représente le groupement :

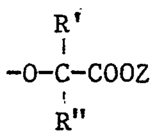 , 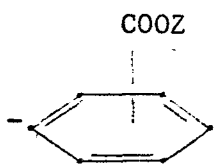 ou -NHCOOZ

dans lequel R' et R" sont tels que définis ci-dessus et Z représente un radical alkyle inférieur, la saponification peut être conduite de telle sorte que le groupe COOZ ne soit pas saponifié. A cet effet, on peut utiliser par exemple, un seul équivalent de base par équivalent d'ester de formule II à une température d'environ 0°C. Il est cependant préférable d'utiliser comme composé de départ un ester de l'acide thiénopyridinonecarboxylique de formule II ci-dessus qui s'hydrolyse dans des conditions telles que le groupement COOZ reste stable. Dans ce but, un groupement E très avantageux est le groupement tertiobutyle qui est aisément clivé par action de l'acide trifluoroacétique. Dans ce cas, l'hydrolyse peut être conduite à la température ambiante en utilisant le même acide trifluoroacétique en tant que solvant de réaction.

Le composé de formule I obtenu après l'hydrolyse peut être transformé en ses sels pharmaceutiquement acceptables selon les techniques connues. Lorsque X représente un groupement :

R'
-O-C-COOZ ,
R"

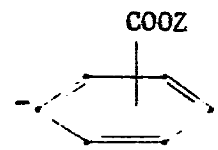

ou -N-COOZ

dans lequel Z est l'hydrogène, la salification peut être effectuée en même temps sur les deux groupes carboxyliques.

Pour préparer un composé de formule I dans laquelle X représente un groupe alkanoyloxy inférieur, le produit provenant de l'hydrolyse du composé de formule II, dans laquelle X est hydroxy, est soumis comme mentionné précédemment, à une réaction d'acylation selon des techniques connues avec un halogénure d'alkanoyle, de préférence le chlorure ou le bromure.

Les composés de formule II utilisés comme composés de départ sont préparés à partir des aldéhydes correspondants de formule :

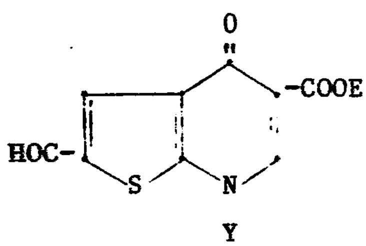

III

dans laquelle Y et E sont tels que définis ci-dessus.

Pour préparer les composés de formule II, où X est hydroxy, on traite la thiénopyridinone de formule III avec le chlorhydrate d'hydroxylamine à la température ambiante dans un solvant organique, tel que l'éthanol.

Pour préparer les composés de formule II, où X est un groupement de formule :

R'
-O-C-W
R"

ou un groupement alkoxy inférieur non substitué ou substitué par un groupement hydroxy, alkoxy inférieur, di-(alkyleinférieur)amino, pipéridino, pyrrolidino, morpholino ou N-(alkyle inférieur)pipérazino, on traite les composés de formule II, où X est hydroxy, avec un chlorure, bromure ou iodure d'alkyle inférieur dont le radical alkyle est non substitué ou substitué comme indiqué ci-dessus ou un halogénure de formule générale :

R'
|
Hal-C-W
|
R"

dans laquelle Hal est le chlore, le brome ou l'iode et R' , R" et W sont tels que définis précédemment en présence d'un agent basique, tel qu'un carbonate de métal alcalin comme le carbonate de sodium ou de potassium ou un bicarbonate alcalin comme le bicarbonate de sodium ou de potassium.

Pour préparer les composés de formule II, dans laquelle X représente un groupement :

R'
|
-O-C-COOZ
|
R"

dans lequel R', R" sont tels que définis ci-dessus et Z représente un radical alkyle inférieur, on traite les composés de formule II, où X est hydroxy, avec un composé de formule générale :

R'
|
Hal-C-COOZ'
|
R"

dans laquelle Hal est le chlore, le brome ou l'iode, R' et R" sont tels que définis ci-dessus et Z' est un groupement alkyle inférieur, en présence d'une base, telle que le carbonate de sodium ou de potassium, l'hydroxyde de sodium ou de potassium ou la triéthylamine.

Le produit ainsi obtenu peut être hydrolysé selon les techniques classiques. Par saponification, on provoque cependant le clivage simultané des substituants Z' et E et la formation de composés de formule I, dans laquelle X représente un groupement :

R'
|
-O-C-COOZ
|
R"

dans lequel Z est l'hydrogène.

Par conséquent, pour obtenir les composés de formule II, dans laquelle X représente un groupement :

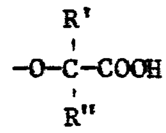

tel que défini précédemment, on effectuera la saponification dans des conditions douces de manière à ne pas saponifier le groupement COOE. Si le groupement Z' est convenablement choisi, l'hydrolyse peut être effectuée d'une façon sélective. Dans ce but, un groupement Z' avantageux est le groupement tertiobutyle qui est clivé facilement par action de l'acide trifluoroacétique.

Pour préparer les composés de formule II, dans laquelle X représente un groupement :

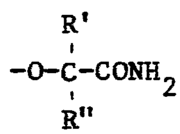

où R' et R" sont tels que définis ci-dessus, on transforme, en leurs dérivés fonctionnels sur l'acide carboxylique libre, les composés correspondants de formule II, dans laquelle X représente un groupement :

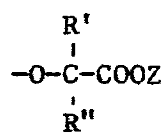

dans lequel R' et R" sont tels que définis ci-dessus et Z est l'hydrogène, ces composés ayant été préparés comme décrit ci-dessus et on traite lesdits dérivés fonctionnels par l'ammoniac gazeux.

Comme dérivé fonctionnel, on peut utiliser un anhydride mixte, tel que formé <u>in situ</u>, par action du chloroformiate d'isobutyle, un ester actif, tel que l'ester de p-nitrophényle ou l'anhydride symétrique formé par action d'un carbodiimide, tel que le cyclohexylcarbodiimide.

Pour préparer les composés de formule II, dans laquelle X représente un groupement :

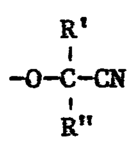

où R' et R" sont tels que définis ci-dessus, on traite un composé de formule

II, dans laquelle X est hydroxy avec un composé de formule :

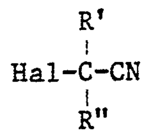

dans laquelle R' et R" sont tels que définis ci-dessus et, Hal est un atome de chlore, de brome ou d'iode, en présence d'une base, telle que le carbonate de sodium ou de potassium, l'hydroxyde de sodium ou de potassium ou la triéthylamine.

Pour préparer les composés de formule II, dans laquelle X représente un radical phényle non substitué ou substitué par un atome de fluor, de chlore, de brome ou d'iode ou par un groupement hydroxy, alkoxy inférieur ou nitro, on traite, au reflux, la thiénopyridinone de formule III avec l'aniline ou une aniline dont le radical phényle est substitué comme indiqué ci-dessus.

Pour préparer les composés de formule II dans laquelle X représente un groupement :

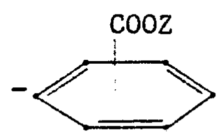

dans lequel Z est tel que défini ci-dessus, on traite au reflux la thiénopyridinone de formule III, avec une aniline de formule générale :

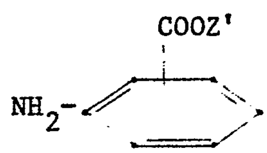

dans laquelle Z' est tel que défini ci-dessus.

Le produit ainsi obtenu peut être hydrolysé selon les techniques classiques. Par saponification, on provoque cependant le clivage simultané des substituants Z' et E et la formation de composés de formule I, dans laquelle X représente un groupement :

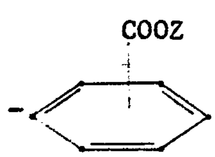

dans lequel Z est l'hydrogène.

Par conséquent, pour obtenir les composés de formule II, dans laquelle X représente un groupement :

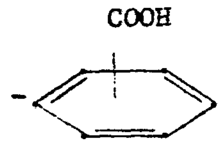

la saponification est effectuée dans des conditions douces de manière à ne pas saponifier le groupement COOE. Si le groupement Z' est convenablement choisi, l'hydrolyse peut être effectuée d'une façon sélective.

Dans ce but, un groupement Z' avantageux est le groupement tertiobutyle qui est clivé facilement par action de l'acide trifluoroacétique.

Pour préparer les composés de formule II dans laquelle X représente un groupement amino ou (alkyle inférieur)amino, on traite la thiénopyridinone de formule III avec l'hydrazine ou une (alkyle inférieure) hydrazine en présence d'une base par exemple l'hydroxyde de sodium ou de potassium.

Pour préparer les composés de formule II dans laquelle X représente un groupement :

$$-NH-COOZ$$

dans lequel Z est tel que défini précédemment, on traite la thiénopyridinone de formule III en présence d'une base par exemple l'hydroxyde de sodium ou de potassium avec un carbazate d'alkyle inférieur de formule générale :

$$NH_2-NH_2-COOZ'$$

dans laquelle Z' est tel que défini précédemment.

Le produit ainsi obtenu peut être hydrolysé selon les techniques classiques. Par saponification, on provoque cependant le clivage simultané des substituents Z' et E et la formation de composés de formule I, dans laquelle X représente un groupement :

$$-NH-COOZ$$

dans lequel Z est l'hydrogène.

Par conséquent, pour obtenir les composés de formule II dans laquelle X représente un groupement :

-NH-COOH

on effectuera la saponification dans des conditions douces de manière à ne pas saponifier le groupement COOE. Si le groupement Z' est convenablement choisi, l'hydrolyse peut être effectuée d'une façon sélective. Dans ce but, un groupement Z' avantageux est le groupement tertiobutyle qui est clivé facilement par action de l'acide trifluoroacétique.

Selon une variante, on prépare les composés de formule I dans laquelle X représente un groupement hydroxy, amino, (alkyle inférieur)amino, alkoxy inférieur ou (alkanoyl inférieur)oxy tel que défini dans la formule I ou un groupement de formule :

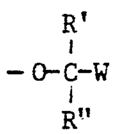 , 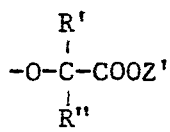 ou -NH-COOZ'

dans lequel R', R", W et Z' sont tels que définis précédemment, en hydrolysant d'abord les composés de formule III selon les techniques classiques de saponification, par chauffage au reflux en présence d'hydroxyde de sodium ou de potassium, puis en acidifiant avec un acide minéral tel que l'acide chlorhydrique.

L'aldéhyde ainsi obtenu de formule générale :

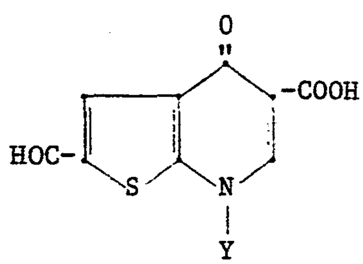

IV

dans laquelle Y est tel que défini ci-dessus, est alors traité, en présence d'une base par exemple en présence d'hydroxyde de sodium ou de potassium, avec l'hydroxylamine, l'hydrazine, une (alkyle inférieure) hydrazine, une (alkyle inférieure) oxyamine, une (alkanoyle inférieure) oxyamine ou un composé de formule générale :

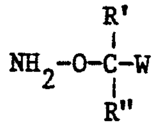 , 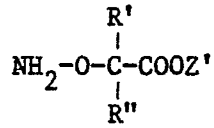 ou $NH_2$-NH-COOZ'

selon la méthode décrite précédemment, puis acidifié avec un acide minéral tel que l'acide chlorhydrique.

Le composé de formule I ainsi obtenu peut alors être transformé en ses sels pharmaceutiquement acceptables.

L'ensemble des procédés décrits ci-dessus permet de préparer les composés de formule I sous forme de mélanges d'isomères syn et anti. Ces isomères individuels peuvent être obtenus, selon des méthodes classiques, par exemple en les séparant de leur mélange, par exemple par chromatographie, cristallisation fractionnée, etc...

D'une autre manière, les isomères individuels syn et anti des composés de formule I peuvent être préparés par hydrolyse des isomères individuels syn et anti des composés correspondants de formule II selon la méthode décrite précédemment pour la préparation des mélanges d'isomères de formule I.

Les composés III et IV sont nouveaux et utiles en tant qu'intermédiaires de synthèse.

Ainsi, la présente invention a pour objet, selon un autre de ses aspects, des composés de formule générale :

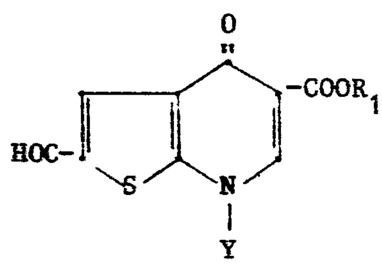

V

dans laquelle Y et $R_1$, qui peuvent être identiques ou différents, représentent chacun l'hydrogène ou un radical alkyle inférieur.

Les composés de formule V sont préparés, selon un autre aspect de la présente invention, à partir des dérivés de thiophène de formule générale :

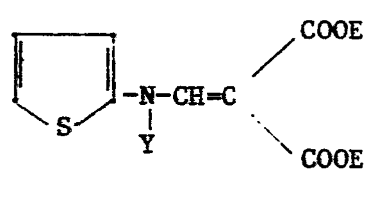

VI

dans laquelle Y et E sont tels que définis ci-dessus, que l'on soumet à une

réaction avec l'oxychlorure de phosphore et le N-méthylformanilide pour obtenir un aldéhyde de formule générale :

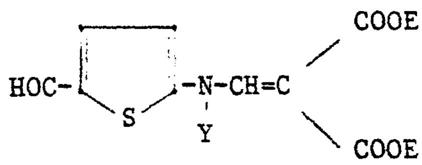

VII

dans laquelle Y et E sont tels que définis ci-dessus.

On effectue ensuite une cyclisation thermique de l'aldéhyde de formule VII en présence d'acide polyphosphorique pour obtenir, après décomposition, les aldéhydes de formule III qui peuvent, si nécessaire, être saponifiés comme décrit ci-dessus.

Les composés de formule VI peuvent être obtenus, lorsque Y représente hydrogène, selon la méthode décrite dans Eur. J. Med. Chem. 1978, 13 (3), 265-269. Le composé de formule VI, dans laquelle Y est hydrogène et E est éthyle, est décrit dans la publication ci-dessus.

Lorsque, dans la formule VI, Y représente alkyle inférieur, les produits concernés peuvent être préparés en faisant réagir un (thiényl-2)aminomalonate de diéthyle avec un p-toluènesulfonate d'alkyle inférieur en présence d'un carbonate de métal alcalin.

Les composés de formule VII ci-dessus sont nouveaux et représentent un objet de la présente invention, selon un autre de ses aspects.

Bien que de nombreux composés soient connus et utilisés comme agents antibactériens, ceux-ci ne présentent pas toujours le degré d'intérêt voulu concernant leur efficacité, leur spectre antibactérien ou leur toxicité.

La recherche de nouveaux composés bactéricides reste,par conséquent, d'un puissant intérêt.

On connait déjà des dérivés de thiénopyridinone doués de propriétés bactéricides. A cet égard, on peut citer le brevet belge No. 858.479 se rapportant notamment à des acides alkyl-7 carboxy-2 dihydro-4,7 oxo-4 thiéno[2,3-b]pyridine carboxylique-5.

Des dérivés bactéricides de thiénopyridinone figurent également dans la demande de brevet japonais No. 51-100092 qui décrit entre autres des acides alkyl-7 dihydro-4,7 oxo-4 thiéno[2,3-b]pyridine carboxylique-5

substitués en position 2 par un radical nitro.

Or, on a trouvé selon l'invention que la fixation, en position 2 d'acides alkyl-7 dihydro-4,7 oxo-4 thiéno[2,3-b]pyridine carboxylique-5 d'un groupement imino, oxyimino ou hydrazono substitué ou non donne naissance à des composés possédant de très intéressantes propriétés bactéricides. Ces propriétés se révèlent, en outre, supérieures à celles présentées par des acides alkyl-7 dihydro-4,7 oxo-4 thiéno[2,3-b]pyridine carboxylique-5 connus notamment des acides alkyl-7 carboxy-2 ou nitro-2 dihydro-4,7 oxo-4 thiéno[2,3-b]pyridine carboxylique-5.

Les composés de formule I ci-dessus et leurs sels pharmaceutiquement acceptables se sont révélés doués d'importantes propriétés bactéricides vis-à-vis d'un nombre élevé de souches pathogènes, à savoir des bactéries gram-positives, gram-négatives et d'autres micro-organismes. En outre, la toxicité présentée par les composés de l'invention s'est montrée compatible avec leur utilisation à des fins thérapeutiques.

A cet effet, les composés de l'invention peuvent être utilisés, à la dose journalière de 10 à 100 mg/kg, dans les infections bactériennes chez les mammifères où interviennent par exemple Escherichia coli, Proteus, Klebsiella, Salmonella, Shigella, Serratia ou Enterobacter.

Ainsi, la présente invention a également pour objet des compositions pharmaceutiques notamment pour le traitement des infections bactériennes contenant, en tant que principe actif, un composé de formule I ci-dessus ou un de ses sels pharmaceutiquement acceptables.

Des tests effectués <u>in vitro</u> suivant le protocole expérimental ci-dessous ont permis de mettre en évidence l'activité antibactérienne en question.

A cet effet, on a déterminé les C.M.I. c'est-à-dire les concentrations minimales inhibitrices de composé à étudier agissant sur le développement des bactéries à 37°C.

On a procédé sur milieu gélosé de MUELLER HINTON à pH=7,4 par la méthode des dilutions sériées en utilisant un inoculum bactérien de $10^4$ unités formant colonies, inoculum déposé sur la gélose à l'aide d'un ensemenceur multiple.

On a utilisé le composé à étudier selon une gamme de concentrations de 100 à 0,05 µg/ml en partant de solutions mères titrant 1000 µg/ml, toutes les dilutions ultérieures ayant été réalisées dans de l'eau distillée.

On a consigné ci-après les résultats obtenus avec les composés suivants de l'invention :

Acide dihydro-4,7 éthyl-7 hydroxyimino-2 oxo-4 thiéno[2,3-b]pyridine carboxylique-5 (Composé A)

Acide dihydro-4,7 éthyl-7 hydroxyimino-2 oxo-4 thiéno[2,3-b]pyridine carboxylique-5, isomère anti (Composé B)

Acide dihydro-4,7 éthyl-7 hydroxyimino-2 oxo-4 thiéno[2,3-b]pyridine carboxylique-5, isomère syn (Composé C)

Acide dihydro-4,7 éthyl-7 méthoxyimino-2 oxo-4 thiéno[2,3-b]pyridine carboxylique-5 (Composé D)

Acide dihydro-4,7 éthyl-7 (N,N-diméthylamino-3 propoxy)imino-2 oxo-4 thiéno[2,3-b]pyridine carboxylique-5 (Composé E)

Acide dihydro-4,7 éthyl-7 N-(hydroxy-2 phényl)imino-2 oxo-4 thiéno[2,3-b] pyridine carboxylique-5 (Composé F)

Acide dihydro-4,7 éthyl-7 N-(méthoxy-4 phényl)imino-2 oxo-4 thiéno[2,3-b] pyridine carboxylique-5 (Composé G)

Acide dihydro-4,7 éthyl-7 méthoxyimino-2 oxo-4 thiéno[2,3-b]pyridine carboxylique-5, isomère anti (Composé H)

Acide dihydro-4,7 éthyl-7 oxo-4 tertiobutoxyimino-2 thiéno[2,3-b]pyridine carboxylique-5 (Composé I)

Acide dihydro-4,7 éthyl-7 N-phénylimino-2 oxo-4 thiéno[2,3-b]pyridine carboxylique-5 (Composé J)

A titre de comparaison, on a effectué des tests similaires avec deux dérivés connus de thiénopyridinone à savoir l'acide dihydro-4,7 éthyl-7 carboxy-2 oxo-4 thiéno[2,3-b]pyridine carboxylique-5 (Composé X) et l'acide dihydro-4,7 éthyl-7 nitro-2 oxo-4 thiéno[2,3-b]pyridine carboxylique-5 (Composé Y).

Tableau

(voir page 14)

Ces résultats montrent que les composés de l'invention se révèlent supérieurs aux composés connus.

TABLEAU

CMI (µg/ml)

| Souches | A | B | C | D | E | F | G | H | I | J | X | Y |
|---|---|---|---|---|---|---|---|---|---|---|---|---|
| | COMPOSES | | | | | | | | | | | |
| Staphylococcus aureus 53154 IP | 25 | 12,5 | 12,5 | 1,56 | 6,25 | 100 | > 100 | > 50 | > 50 | > 100 | > 100 | > 100 |
| Streptococcus faecalis 5855 IP | > 100 | > 100 | > 100 | > 100 | 100 | > 100 | > 100 | > 100 | > 100 | > 100 | > 100 | > 100 |
| Bacillus subtilis ATCC 6633 | - | 6,25 | 6,25 | 0,78 | 6,25 | 6,25 | 6,25 | 0,8 | 1,56 | - | - | - |
| Escherichia coli 54127 IP | 3,12 | 3,12 | 12,5 | 0,78 | 6,25 | 1,56 | 1,56 | 1,56 | > 25 | 3,12 | > 100 | 3,12 |
| Escherichia coli SOL RL 90 | 6,25 | 3,12 | 3,12 | 3,12 | 25 | 3,12 | 3,12 | 6,25 | > 50 | 6,25 | > 100 | 6,25 |
| Citrobacter freundii GN 346 | 6,25 | 3,12 | 3,12 | 3,12 | 12,5 | 6,25 | 6,25 | 6,25 | > 25 | 12,5 | > 100 | 12,5 |
| Proteus vulgaris RL 99 bis | 1,56 | 0,80 | 12,5 | 0,78 | 12,5 | 3,12 | 1,56 | 1,56 | > 50 | 3,12 | > 100 | 3,12 |
| Proteus mirabilis ATCC 21100 | 1,56 | 1,56 | 12,5 | 0,78 | 3,12 | 3,12 | 6,25 | 0,8 | > 25 | 12,5 | > 100 | 12,5 |
| Proteus morganii 1510 | 1,56 | 0,40 | 0,40 | 3,12 | 25 | 6,25 | 0,78 | 0,8 | > 25 | 3,12 | > 100 | 3,12 |
| Providencia stuartii 5158 | 6,25 | 6,25 | 12,5 | 3,12 | 25 | 12,5 | 12,5 | 6,25 | > 50 | 12,5 | > 100 | 12,5 |
| Serratia liquefaciens 376 | 100 | 50 | 50 | 50 | 50 | > 100 | 100 | > 25 | > 25 | > 100 | > 100 | > 100 |
| Klebsiella pneumoniae ATCC 10031 | 1,56 | 1,56 | 12,5 | 0,2 | 3,12 | 12,5 | 12,5 | 0,4 | 3,12 | 50 | > 100 | 50 |
| Klebsiella pneumoniae R 30 | 6,25 | 6,25 | 6,25 | 3,12 | 25 | 12,5 | 12,5 | 6,25 | > 25 | 25 | > 100 | 25 |
| Enterobacter cloacae P 99 | 6,25 | 6,25 | 3,12 | 1,56 | 12,5 | 6,25 | 6,25 | 3,12 | > 25 | 50 | > 100 | 50 |
| Enterobacter hafniae RO 46 | 1,56 | 3,12 | 12,5 | 1,56 | 6,25 | 1,56 | 1,56 | 3,12 | > 25 | 3,12 | > 100 | 3,12 |
| Shigella sonnei R 018 | 1,56 | 0,80 | 0,80 | 1,56 | 25 | 6,25 | 6,25 | 3,12 | > 50 | 3,12 | > 100 | 3,12 |
| Salmonella typhimurium A 222 IP | 6,25 | 3,12 | 3,12 | 3,12 | 25 | 3,12 | 3,12 | 12,5 | > 50 | 6,25 | > 100 | 6,25 |
| Salmonella typhi ATCC 6539 | 6,25 | 6,25 | 3,12 | 3,12 | 25 | 3,12 | 6,25 | 25 | > 25 | 25 | > 100 | 25 |
| Bordetella bronchiseptica ATCC 4617 | > 100 | > 100 | > 100 | 12,5 | > 100 | > 100 | > 100 | > 25 | > 25 | > 100 | > 100 | > 100 |
| Pseudomonas aeruginosa 8203 | 50 | 100 | > 100 | > 100 | > 100 | > 100 | > 100 | > 25 | > 25 | > 100 | > 100 | > 100 |
| Acinetobacter calcoaceticus C 1733 | > 100 | > 100 | > 100 | > 100 | > 100 | 100 | > 100 | > 25 | > 25 | > 100 | > 100 | > 100 |

Les compositions thérapeutiques de l'invention peuvent être présentées sous toutes formes convenant à l'administration en thérapie humaine ou vétérinaire.

Pour ce qui concerne l'unité d'administration, celle-ci peut prendre la forme, par exemple, d'un comprimé dragéifié ou non, d'une capsule, d'une gélule, d'une poudre, d'un granulé, d'une suspension ou d'un sirop pour l'administration orale, d'un suppositoire pour l'administration rectale, d'une solution stérile ou suspension pour l'administration parentérale ou d'une forme d'administration intra-utérine ou intramammaire.

La quantité totale de principe actif peut varier entre 100 et 5000 mg par unité d'administration, ledit principe actif pouvant être seul ou en mélange avec les véhicules ou excipients pharmaceutiques appropriés tels que, par exemple, eau distillée, amidons, talc, stéarate de magnésium, polyvinylpyrrolidone, acide alginique, silice colloïdale, chlorure de sodium, dioxyde de titane, beurre de cacao, agents édulcorants et similaires.

Les exemples suivants illustrent l'invention sans toutefois la limiter.

## PREPARATIONS

a) N-éthyl-N-(thiényl-2)-aminoéthylènemalonate de diéthyle

On dissout 18g (0,067 mole) de (thiényl-2)-aminométhylènemalonate de diéthyle dans 150 ml d'acétone avec 11g (0,08 mole) de carbonate de potassium. On porte au reflux pendant 3h et on filtre le précipité qui s'est formé. Après lavage à l'éther éthylique, on sèche. On dissout le solide obtenu dans une solution de 150 ml de N,N-diméthylformamide contenant 13g (0,066 mole) de p-toluènesulfonate d'éthyle puis on porte au reflux pendant 10h. Après refroidissement, on élimine le solvant sous vide. On reprend le résidu dans le chloroforme, on lave à l'eau et on sèche. On évapore à sec la phase organique et on dissout le résidu dans l'hexane chaud. Après précipitation à froid, on essore le précipité obtenu sur verre fritté.

On obtient ainsi le N-éthyl-N-(thiényl-2)-aminoéthylènemalonate de diéthyle avec un rendement de 80%.

P.F. : 41-42°C

C.C.M. : { CH2Cl2 : 6 Rf : 0,8
{ éther : 4

b) N-éthyl-N-(formyl-5 thiényl-2)-aminoéthylènemalonate de diéthyle

Au réactif de VILSMEIR (N-méthylformanilide et oxychlorure de phosphore), on ajoute lentement sous agitation 5g de N-éthyl-N-(thiényl-2)-aminométhylènemalonate de diéthyle dissous dans 20ml de dichloro-1,2 éthane. On maintient l'agitation pendant 5h puis on verse le mélange réactionnel sur 100g de glace contenant 5g d'acétate de sodium de manière à neutraliser la solution. On extrait l'aldéhyde puis on lave la phase organique à l'eau. On sèche et évapore à sec. On reprend ensuite le résidu dans l'hexane chaud et on filtre le précipité sur verre fritté.

On obtient ainsi le N-éthyl-N-(formyl-5 thiényl-2)-aminoéthylènemalonate de diéthyle avec un rendement de 70%.

P.F. : 45°C (hexane)

EXEMPLE I

Ester éthylique de l'acide dihydro-4,7 éthyl-7 formyl-2 oxo-4 thiéno[2,3-b] pyridine carboxylique-5

Sous agitation, on chauffe à 120°C 20g d'acide polyphosphorique auquel on additionne 1,5g de N-éthyl-N-(formyl-5 thiényl-2)-aminoéthylènemalonate de diéthyle. On maintient l'agitation et le chauffage pendant 30 min. Après refroidissement, à température ambiante, on décompose par 100g de glace pilée. Après homogénéisation, on amène la solution refroidie à pH 3-4 avec du carbonate de sodium puis on extrait au chloroforme. On lave la phase organique à l'eau, sèche et évapore à sec.

On obtient ainsi 1g d'ester éthylique de l'acide dihydro-4,7 éthyl-7 formyl-2 oxo-4 thiéno[2,3-b]pyridine carboxylique-5 avec un rendement de 78%.

P.F. : 205°C (éthanol)

EXEMPLE II

Acide dihydro-4,7 éthyl-7 formyl-2 oxo-4 thiéno[2,3-b]pyridine carboxylique-5

On chauffe au bain-marie pendant quelques minutes un mélange de 3 parties de dihydro-4,7 éthyl-7 formyl-2 oxo-4 thiéno[2,3-b]pyridine carboxylate-5 d'éthyle et 100 parties en volume d'une solution à 10% d'hydroxyde de potassium dans de l'éthanol. On laisse le mélange 6h à la température ambiante, on filtre le précipité, on le lave avec de l'éthanol et on ajoute de l'acide chlorhydrique dilué. On obtient ainsi l'acide dihydro-4,7 éthyl-7 formyl-2 oxo-4 thiéno[2,3-b]pyridine carboxylique-5.

EXEMPLE III

Ester éthylique de l'acide dihydro-4,7 éthyl-7 hydroxyimino-2 oxo-4 thiéno [2,3-b]pyridine carboxylique-5

On dissout 0,5g d'ester éthylique de l'acide dihydro-4,7 éthyl-7 formyl-2 oxo-4 thiéno[2,3-b]pyridine carboxylique-5 dans 50ml d'éthanol chaud. On refroidit la solution à température ambiante, puis on ajoute 0,2g de chlorhydrate d'hydroxylamine suivi de 2ml d'une solution d'hydroxyde de sodium à 5%. Après filtration, on obtient l'ester éthylique de l'acide dihydro-4,7 éthyl-7 hydroxyimino-2 oxo-4 thiéno[2,3-b]pyridine carboxylique-5 avec un rendement de 80%.

EXEMPLE IV

Ester éthylique de l'acide dihydro-4,7 éthyl-7 hydroxyimino-2 oxo-4 thiéno [2,3-b]pyridine carboxylique-5

On dissout à chaud 0,8g (2,8 mmoles) d'ester éthylique de l'acide dihydro-4,7 éthyl-7 formyl-2 oxo-4 thiéno[2,3-b]pyridine carboxylique-5 dans 50ml d'éthanol. Après refroidissement de la solution jusqu'à la température ambiante, on ajoute 0,25g (3,6 mmoles) de chlorhydrate d'hydroxylamine dissous dans 1ml d'eau. A cette solution, on ajoute une solution diluée d'hydroxyde de sodium jusqu'à obtenir un pH voisin de 7. On maintient l'agitation durant 2h à température ambiante.

On place le mélange réactionnel au congélateur de manière à obtenir la précipitation de l'ester éthylique de l'acide dihydro-4,7 éthyl-7 hydroxyimino-2 oxo-4 thiéno[2,3-b]pyridine carboxylique-5 qui est filtré.
Rendement : 70%.

La C.C.M. sur gel de silice (éluant : chloroforme/éthanol 18:2) montre 2 spots : Rf = 0,43 et Rf = 0,36.

On sépare les deux isomères obtenus par recristallisation dans un mélange de N,N-diméthylformamide et de méthanol.

Le composé de Rf = 0,43 est cristallin dans le N,N-diméthylformamide à température ambiante, le composé de Rf = 0,36 est cristallin dans le méthanol froid.

L'étude des spectres de RMN montre que l'isomère de Rf = 0,43 est l'isomère anti, celui de Rf = 0,36 est l'isomère syn.

Isomère anti

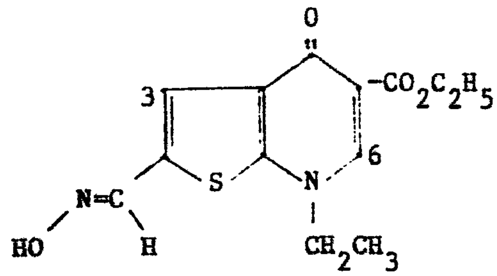

P.F. : 288-290°C (décomposition)

R.M.N.

| | | | | |
|---|---|---|---|---|
| 1,25 | ppm | triplet | | |
| 1,4 | ppm | triplet | | |
| 4,2 | ppm | quartet | | |
| 7,65 | ppm | singulet | CH | $H_3$ |
| 8,5 | ppm | singulet | CH | oxime |
| 8,6 | ppm | singulet | CH | $H_6$ |

Isomère syn

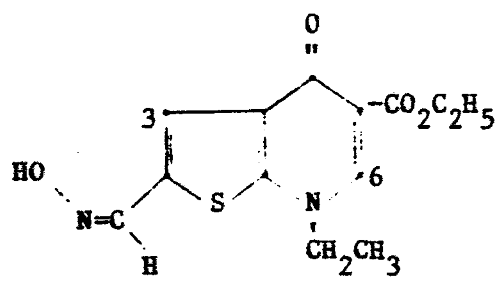

P.F. : 315-318°C

R.M.N.

7,8 ppm singulet CH oxime

EXEMPLE V

Ester éthylique de l'acide dihydro-4,7 éthyl-7 carbométhoxyimino-2 oxo-4 thiéno[2,3-b]pyridine carboxylique-5

On dissout 0,3g (1,1 mmole) d'ester éthylique de l'acide dihydro-4,7 éthyl-7 formyl-2 oxo-4 thiéno[2,3-b]pyridine carboxylique-5 dans 40ml d'éthanol chaud. On refroidit la solution à 20°C, puis on y ajoute 0,14g (1,1 mmole) de chlorhydrate de carboxyméthoxyamine dissous dans 10ml d'eau. On maintient le mélange réactionnel sous agitation pendant 1h à température ambiante.

On suit l'avancement de la réaction par C.C.M. (éluant : chloroforme/

éthanol 19:1).

On obtient ainsi une solution contenant l'ester éthylique de l'acide dihydro-4,7 éthyl-7 carbométhoxyimino-2 oxo-4 thiéno[2,3-b]pyridine carboxylique-5.

EXEMPLE VI

Ester éthylique de l'acide dihydro-4,7 éthyl-7 (méthoxy-4 phényl)-imino-2 oxo-4 thiéno[2,3-b]pyridine carboxylique-5.

On chauffe au reflux du méthanol pendant 3 heures, un mélange de 0,2g (0,72 mmole) de l'ester éthylique de l'acide dihydro-4,7 éthyl-7 formyl-2 oxo-4 thiéno[2,3-b]pyridine carboxylique-5 et 0,09g (0,72 mmole) de p-méthoxy-aniline dans 20 ml de méthanol.

Après refroidissement du mélange réactionnel, on recueille l'imine attendue par filtration avec un rendement de 50%.
C.C.M. : Rf = 0,28 (éluant : chloroforme/éthanol 19:1).

A partir de produits intermédiaires tels que préparés ci-dessus, on a obtenu les composés suivants de formule I :

EXEMPLE 1

Acide dihydro-4,7 éthyl-7 hydroxyimino-2 oxo-4 thiéno[2,3-b]pyridine carboxylique-5.

On chauffe au reflux pendant 1h, 1g d'ester éthylique, de l'acide dihydro-4,7 éthyl-7 hydroxyimino-2 oxo-4 thiéno[2,3-b]pyridine carboxylique-5 dans 30 ml d'hydroxyde de sodium à 10%. Après refroidissement, on filtre la solution et on acidifie avec de l'acide chlorhydrique à 10%. Après filtration, on obtient l'acide dihydro-4,7 éthyl-7 hydroxyimino-2 oxo-4 thiéno [2,3-b]pyridine carboxylique-5 avec un rendement de 80%.
P.F. : 315-317°C (N,N-diméthylformamide).

EXEMPLE 2

Isomère anti de l'acide dihydro-4,7 éthyl-7 hydroxyimino-2 oxo-4 thiéno [2,3-b]pyridine carboxylique-5

On dissout 0,125g (0,42 mmole) de l'isomère anti de l'ester éthylique de l'acide dihydro-4,7 éthyl-7 hydroxyimino-2 oxo-4 thiéno[2,3-b]pyridine carboxylique-5 dans 10ml d'eau contenant 0,4g d'hydroxyde de sodium.

On laisse la solution obtenue sous agitation à la température ambiante pendant 1h.

On acidifie à l'acide chlorhydrique concentré, on filtre le précipité obtenu, on lave à l'eau puis à l'alcool. Après séchage, on obtient l'isomère anti de l'acide dihydro-4,7 éthyl-7 hydroxyimino-2 oxo-4 thiéno[2,3-b]pyridine carboxylique-5 avec un rendement de 98%.

P.F. : 332-334°C.

Spectre R.M.N.

L'étude du spectre R.M.N. montre qu'il n'y a pas eu d'isomérisation au cours de la réaction.

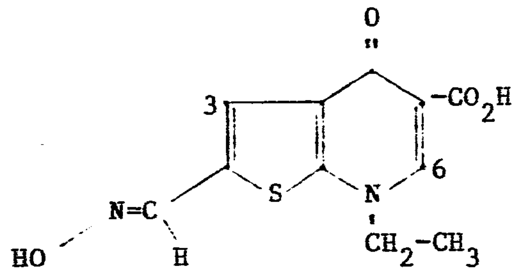

| | | | | |
|---|---|---|---|---|
| 1,5 | ppm | triplet | $CH_3$ | |
| 4,4 | ppm | quartet | $CH_2$ | |
| 7,7 | ppm | singulet | CH | $H_3$ |
| 8,5 | ppm | singulet | CH | oxime |
| 8,9 | ppm | singulet | CH | $H_6$ |

EXEMPLE 3

Isomère syn de l'acide dihydro-4,7 éthyl-7 hydroxyimino-2 oxo-4 thiéno[2,3-b] pyridine carboxylique-5

Le même processus opératoire que celui décrit à l'Exemple 2 fournit, à partir de l'isomère syn de l'ester éthylique de l'acide dihydro-4,7 éthyl-7 hydroxyimino-2 oxo-4 thiéno[2,3-b]pyridine carboxylique-5, l'isomère syn de l'acide dihydro-4,7 éthyl-7 hydroxyimino-2 oxo-4 thiéno[2,3-b]pyridine carboxylique-5 avec un rendement de 58%.

P.F. : 330-335°C.

Spectre R.M.N.

L'étude du spectre R.M.N. montre qu'il n'y a pas eu d'isomérisation au cours de la réaction.

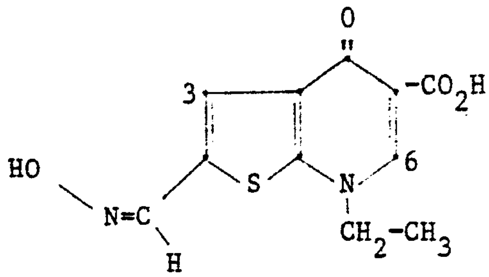

| | | | | |
|---|---|---|---|---|
| 1,5 | ppm | triplet | $CH_3$ | |
| 4,5 | ppm | triplet | $CH_2$ | |
| 8 | ppm | singulet | CH | $H_3$ |
| 8,15 | ppm | singulet | CH | oxime |
| 9,05 | ppm | singulet | CH | $H_6$ |

EXEMPLE 4

Acide dihydro-4,7 éthyl-7 carboxyméthoxyimino-2 oxo-4 thiéno[2,3-b]pyridine carboxylique-5

On ajoute à la solution obtenue à l'Exemple V contenant l'ester éthylique de l'acide dihydro-4,7 éthyl-7 carboxyméthoxyimino-2 oxo-4 thiéno [2,3-b]pyridine carboxylique-5, 0,15g (3,7 mmoles) d'hydroxyde de sodium dans 5ml d'eau et on laisse sous agitation durant 3h à température ambiante. On filtre le précipité formé, lave à l'éthanol puis à l'éther éthylique. On dissout le produit ainsi recueilli dans 20ml d'eau et on acidifie la solution avec de l'acide chlorhydrique concentré. On filtre le précipité formé et on lave à l'eau puis à l'éthanol. Après sèchage, on obtient l'acide dihydro-4,7 éthyl-7 carboxyméthoxyimino-2 oxo-4 thiéno[2,3-b]pyridine carboxylique-5 avec un rendement de 40%.

P.F. : 249-250°C (N,N-diméthylformamide/éthanol)

C.C.M. (gel de silice) : Rf = 0,57 (éluant : chloroforme/éthanol/eau 10:9,5:0,5).

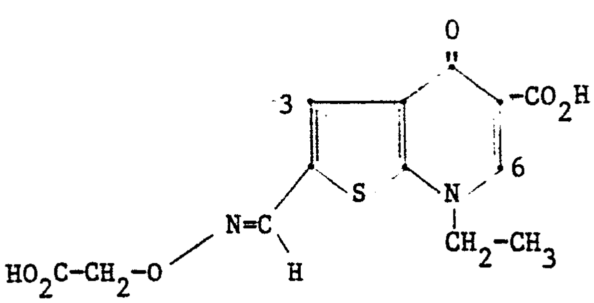

Spectre I.R.

Conforme.

Spectre R.M.N. (DMSO $d_6$)

| | | | | | | |
|---|---|---|---|---|---|---|
| 1,5 | ppm | triplet | $CH_3$ | | | |
| 4,5 | ppm | quartet | $CH_2$ | | | |
| 4,7 | ppm | singulet | $CH_2$ | (isomère anti) | | |
| 4,85 | ppm | singulet | $CH_2$ | (isomère syn) | | |
| 7,9 | ppm | singulet | CH | $H_3$ | } | |
| 8,7 | ppm | singulet | CH | oxime | } | isomère anti |
| 9,0 | ppm | singulet | CH | $H_6$ | } | |

EXEMPLE 5

Acide dihydro-4,7 éthyl-7 (méthoxy-4 phényl)-imino-2 oxo-4 thiéno[2,3-b] pyridine carboxylique-5

On met en solution 0,1g d'ester éthylique de l'acide dihydro-4,7 éthyl-7 (méthoxy-4 phényl)-imino-2 oxo-4 thiéno[2,3-b]pyridine carboxylique-5 obtenu à l'Exemple VI dans 10 ml d'eau contenant 0,1g d'hydroxyde de sodium et 10ml d'éthanol. On agite le mélange à température ambiante jusqu'à hydrolyse totale et on suit l'avancement de la réaction par CCM (éluant : chloroforme/éthanol 19:1). Lorsque la réaction est terminée, on neutralise la solution avec une solution d'acide chlorhydrique dilué. On essore le précipité formé sur verre fritté et on lave à l'eau puis à l'éthanol. On sèche dans un dessicateur et on recueille ainsi 0,08g d'acide dihydro-4,7 éthyl-7 (méthoxy-4 phényl)-imino-2 oxo-4 thiéno[2,3-b]pyridine carboxylique-5 avec un rendement de 90%.

P.F. : 301-303°C (N,N-diméthylformamide).

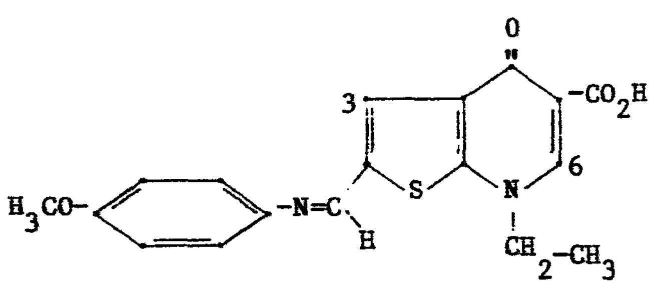

Spectre R.M.N. (DMSO $d_6$, referent : TMS)

| | | | | | |
|---|---|---|---|---|---|
| 1,8 | ppm | triplet | 3H | | |
| 4,0 | ppm | singulet | 3H | | |
| 4,7 | ppm | quartet | 2H | | |
| 7,4 | ppm | massif (4 pics) | 4H | | |
| 8,3 | ppm | singulet | 1H | ($H_3$) | isomère anti |
| 9,2 | ppm | singulet | 2H | ($H_6$ et imine) | |

EXEMPLE 6

Acide dihydro-4,7 éthyl-7 oxo-4 tertiobutoxyimino-2 thiéno[2,3-b]pyridine carboxylique-5

Dans 40ml d'eau contenant 0,1g (2,5 mmoles) d'hydroxyde de sodium, on dissout 0,4g (1,6 mmole) d'acide dihydro-4,7 éthyl-7 formyl-2 oxo-4 thiéno [2,3-b]pyridine carboxylique-5 tel qu'obtenu à l'Exemple II. Lorsque la solution est limpide, on ajoute 0,2g (1,6 mmole) de chlorhydrate de tertiobutoxyamine dissous dans 5ml d'eau puis on maintient la solution obtenue sous agitation pendant 2 heures. On neutralise le milieu à l'acide chlorhydrique dilué puis on essore sur verre fritté le précipité qui se forme. On le lave à l'eau puis à l'éthanol puis on sèche dans un dessicateur.

De cette manière, on recueille 0,4g d'acide dihydro-4,7 éthyl-7 oxo-4 tertiobutoxyimino-2 thiéno[2,3-b]pyridine carboxylique-5 avec un rendement de 80%.

P.F. : 223-224°C (N,N-diméthylformamide)

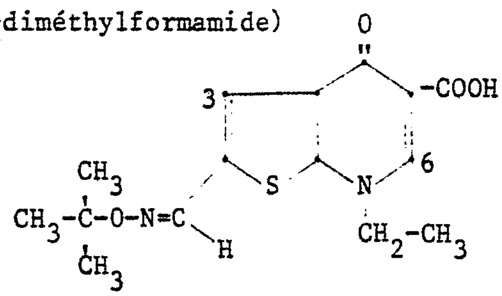

Spectre R.M.N. (DMSO $d_6$, référent : TMS)

L'échantillon contient 70% de l'isomère anti et 30% d'isomère syn.

| | | | | |
|---|---|---|---|---|
| 1,4 | ppm | singulet | $(CH_3)_3$ | isomère anti |
| 1,5 | ppm | singulet | $(CH_3)_3$ | isomère syn |
| 1,8 | ppm | triplet | $CH_3$ | |
| 4,5 | ppm | quartet | $CH_2$ | |
| 7,9 | ppm | singulet | $H_3$ | isomère anti |
| 8,1 | ppm | singulet | $H_3$ | isomère syn |

| | | | | | |
|---|---|---|---|---|---|
| 8,25 | ppm | singulet | H | oxime | isomère syn |
| 8,6 | ppm | singulet | H | oxime | isomère anti |
| 9,05 | ppm | singulet | $H_6$ | | isomère anti |
| 9,1 | ppm | singulet | $H_6$ | | isomère syn |

## EXEMPLE 7

Acide dihydro-4,7 éthyl-7 (carboxyméthoxyamino)-imino-2 oxo-4 thiéno[2,3-b] pyridine carboxylique-5

Dans 50 ml d'eau contenant 0,05g (1,25 mmole) d'hydroxyde de sodium, on dissout 0,2g d'acide dihydro-4,7 éthyl-7 formyl-2 oxo-4 thiéno[2,3-b]pyridine carboxylique-5 préparé comme à l'Exemple II.

Après solubilisation, on ajoute 0,07g (0,8 mmole) de carbazate de méthyle et on chauffe la solution obtenue à 60°C pendant 3h. On refroidit le milieu puis on le neutralise avec de l'acide chlorhydrique dilué.

On essore sur verre fritté le précipité qui s'est formé, on le lave à l'eau puis à l'éthanol. Après sèchage dans un dessicateur, on recueille 0,12g d'acide dihydro-4,7 éthyl-7 (carboxyméthoxyamino)-imino-2 oxo-4 thiéno[2,3-b] pyridine carboxylique-5 sous forme d'un produit jaune clair que l'on recristallise dans un mélange N,N-diméthylformamide/éthanol.

P.F. : > 315°C

Rendement : 47%.

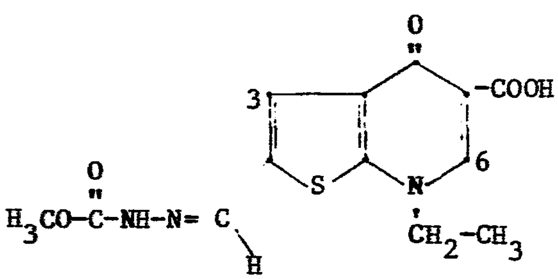

Spectre R.M.N. (DMSO $d_6$, référent : TMS)

L'échantillon contient 100% de l'isomère anti

| | | | | |
|---|---|---|---|---|
| 1,5 | ppm | triplet | 3H | |
| 3,8 | ppm | singulet | 3H | |
| 4,5 | ppm | quartet | 2H | |
| 7,8 | ppm | singulet | $H_3$ | isomère anti |
| 8,3 | ppm | singulet | imine | |
| 8,9 | ppm | singulet | $H_6$ | |

Au départ des produits appropriés, on a préparé les composés suivants en utilisant les procédés décrits dans les Exemples précédents :

Acide dihydro-4,7 éthyl-7 méthoxyimino-2 oxo-4 thiéno[2,3-b]pyridine carboxylique-5
P.F. : 244-245°C.

Acide dihydro-4,7 éthyl-7 (N,N-diméthylamino-3 propoxy)-imino-2 oxo-4 thiéno[2,3-b]pyridine carboxylique-5
P.F. : 259-261°C.

Acide dihydro-4,7 éthyl-7 N-(hydroxy-2 phényl)-imino-2 oxo-4 thiéno[2,3-b] pyridine carboxylique-5
P.F. : 320-322°C.

Acide dihydro-4,7 éthyl-7 N-phénylimino-2 oxo-4 thiéno[2,3-b]pyridine carboxylique-5
P.F. : 270-272°C.

Acide dihydro-4,7 éthyl-7 [(diméthyl-1,1 carboxy-1)-méthoxy]-imino-2 oxo-4 thiéno[2,3-b]pyridine carboxylique-5
P.F. : 218-221°C.

Acide dihydro-4,7 éthyl-7 méthoxyimino-2 oxo-4 thiéno[2,3-b]pyridine carboxylique-5,isomère anti
P.F. : 244-245°C.

Acide dihydro-4,7 éthyl-7 (N,N-diisopropylamino-2 éthoxy)-imino-2 oxo-4 thiéno[2,3-b]pyridine carboxylique-5
P.F. : 235-237°C.

Acide dihydro-4,7 éthyl-7 allyloxyimino-2 oxo-4 thiéno[2,3-b]pyridine carboxylique-5
P.F. : 225-227°C.

Acide dihydro-4,7 éthyl-7 éthoxyimino-2 oxo-4 thiéno[2,3-b]pyridine carboxylique-5
P.F. : 220-221°C.

Acide dihydro-4,7 éthyl-7 aminoimino-2 oxo-4 thiéno[2,3-b]pyridine carboxylique-5
P.F. : > 310°C.

EXEMPLE 8

Sel de sodium de l'acide dihydro-4,7 éthyl-7 hydroxyimino-2 oxo-4 thiéno [2,3-b]pyridine carboxylique-5

Dans 40ml d'éthanol contenant 15ml d'une solution aqueuse d'hydroxyde de sodium 0,25 N, on introduit 1 mmole d'acide dihydro-4,7 éthyl-7 hydroxyimino-2 oxo-4 thiéno[2,3-b]pyridine carboxylique-5. On abandonne au repos puis on filtre le précipité formé. On lave avec de l'éthanol puis on sèche dans un dessicateur.

De cette manière, on obtient le sel de sodium de l'acide dihydro-4,7 éthyl-7 hydroxyimino-2 oxo-4 thiéno[2,3-b]pyridine carboxylique-5 avec un rendement de 85%.

EXEMPLE 9

On prépare une composition aqueuse stérile injectable répondant à la formulation suivante :

| | |
|---|---|
| Acide dihydro-4,7 éthyl-7 hydroxyimino-2 oxo-4 thiéno[2,3-b]pyridine carboxylique-5 | 1000 mg |
| Hydroxyde de sodium, eau qs | 10 ml |

pH de la solution : de 11 à 11,5

REVENDICATIONS

1. Dérivés de thiénopyridinone de formule générale :

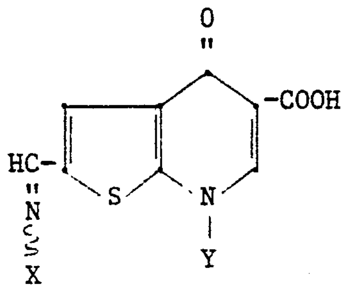 I

dans laquelle Y représente l'hydrogène ou un groupement alkyle inférieur et X représente :

- un groupement hydroxy, alkanoyloxy inférieur, alkoxy inférieur non substitué ou substitué par un groupement hydroxy, alkoxy inférieur, di-(alkyle inférieur) amino, pipéridino, pyrrolidino, morpholino ou N-(alkyle inférieur)pipérazino,
- un groupement de formule :

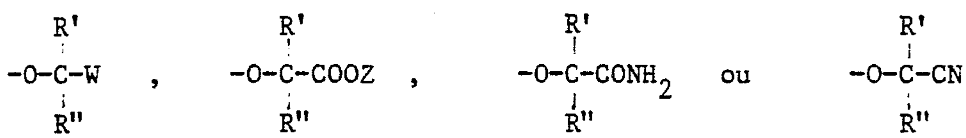

  dans laquelle R' et R", qui sont identiques ou différents, représentent chacun l'hydrogène ou un radical alkyle inférieur, W représente l'hydrogène, un groupement alkyle inférieur ou un groupement vinyle ou éthynyle et Z représente l'hydrogène ou un radical alkyle inférieur,
- un radical phényle non substitué ou substitué par un atome de fluor, de chlore, de brome ou d'iode ou par un groupement hydroxy, alkoxy inférieur, ou nitro,
- un groupement de formule :

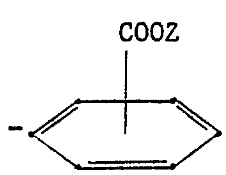

  dans laquelle Z a la même signification que précédemment,
- un groupement amino, (alkyle inférieur)amino ou -NHCOOZ dans lequel Z a la même signification que précédemment ainsi que leurs sels pharmaceutiquement acceptables.

2. Acide dihydro-4,7 éthyl-7 hydroxyimino-2 oxo-4 thiéno[2,3-b]pyridine carboxylique-5 et ses sels pharmaceutiquement acceptables.
3. Acide dihydro-4,7 éthyl-7 hydroxyimino-2 oxo-4 thiéno[2,3-b]pyridine carboxylique-5, isomère anti ainsi que ses sels pharmaceutiquement acceptables.
4. Acide dihydro-4,7 éthyl-7 hydroxyimino-2 oxo-4 thiéno[2,3-b]pyridine carboxylique-5, isomère syn ainsi que ses sels pharmaceutiquement acceptables.
5. Procédé de préparation de dérivés de thiénopyridinone selon la Revendication 1, caractérisé en ce que l'on hydrolyse un ester de l'acide thiénopyridinonecarboxylique de formule générale :

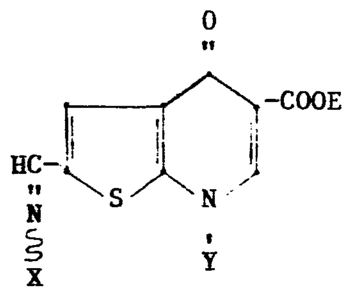

II

dans laquelle X et Y sont tels que définis dans la Revendication 1 et E représente un radical alkyle inférieur et lorsque X est l'hydrogène, l'on soumet, si nécessaire, le produit ainsi obtenu à une réaction d'acylation avec un halogénure d'alkanoyle inférieur et l'on transforme, si on le désire, le produit d'hydrolyse et d'acylation éventuelle en ses sels pharmaceutiquement acceptables.
6. Procédé selon la Revendication 5, caractérisé en ce que, dans le composé de départ de formule II, E représente tertiobutyle et X représente un groupement de formule générale :

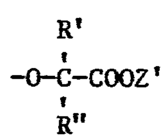

dans lequel R' et R" sont tels que définis dans la Revendication 1 et Z' représente un radical alkyle inférieur et l'hydrolyse est effectuée avec l'acide trifluoroacétique.
7. A titre de produits intermédiaires utiles dans le procédé selon l'une des Revendications 2 ou 3, les composés de formule générale :

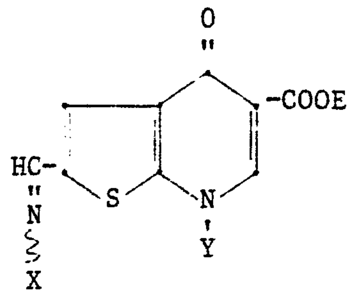

II

dans laquelle Y est tel que défini dans la Revendication 1 et X et E sont tels que définis dans l'une des Revendications 5 ou 6.

8. Procédé selon la Revendication 5, caractérisé en ce que les composés de départ de formule II sont préparés à partir d'un aldéhyde de formule générale :

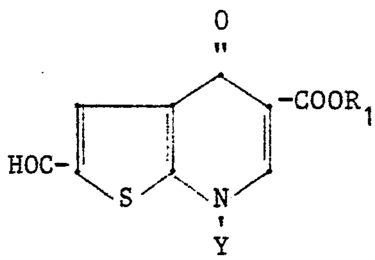

V

dans laquelle Y et $R_1$, qui peuvent être identiques ou différents, représentent chacun l'hydrogène ou un radical alkyle inférieur,

a) par réaction du composé de formule V, dans laquelle $R_1$ est un radical alkyle inférieur, avec le chlorhydrate d'hydroxylamine à la température ambiante dans un solvant organique, suivie, si nécessaire, de :

b1) la réaction, en présence d'un agent basique, du produit obtenu sous a) avec un chlorure, bromure ou iodure d'alkyle inférieur non substitué ou substitué par un groupement hydrox  alkoxy inférieur, di(alkyle inférieur)amino, pipéridino, pyrrolidino, morpholino ou N-(alkyle inférieur)pipérazino ou un composé de formule générale :

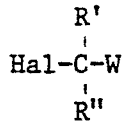

dans laquelle R', R" et W sont tels que définis dans la Revendication 1 et Hal représente chlore, brome ou iode, ou

b2) la réaction du produit obtenu sous a) avec un composé de formule générale :

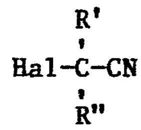

dans laquelle Hal, R' et R" sont tels que définis ci-dessus, en présence d'une base, ou

b3) la réaction du produit obtenu sous a) avec un composé de formule générale :

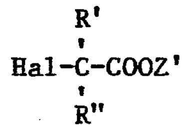

dans laquelle Hal, R' et R" sont tels que définis ci-dessus et Z' représente un radical alkyle inférieur et de l'hydrolyse sélective éventuelle du radical Z' ; et, éventuellement,

b4) de la transformation de l'acide obtenu sous b3), après hydrolyse sélective, en un de ses dérivés fonctionnels et réaction de ce dernier avec de l'ammoniac gazeux, ou

c) par réaction du composé de formule V dans laquelle $R_1$ est un radical alkyle inférieur, avec l'aniline ou une aniline dont le radical phényle est substitué par un atome de fluor, de chlore, de brome ou d'iode ou par un groupement hydroxy, alkoxy inférieur ou nitro, ou

d) par réaction du composé de formule V dans laquelle $R_1$ est un radical alkyle inférieur, avec une aniline de formule générale :

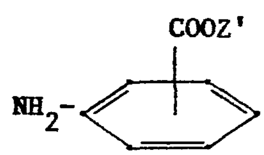

dans laquelle Z' est tel que défini ci-dessus et de l'hydrolyse sélective éventuelle du radical Z', ou

e) la réaction du composé de formule V dans laquelle $R_1$ est un radical alkyle inférieur, avec l'hydrazine ou une (alkyle inférieur)hydrazine en présence d'un agent basique, ou

f) la réaction en présence d'un agent basique du composé de formule V dans laquelle $R_1$ est un radical alkyle inférieur, avec un carbazate d'alkyle inférieur de formule générale :

$$NH_2\text{-}NH\text{-}COOZ'$$

dans laquelle Z' est tel que défini ci-dessus et de l'hydrolyse sélective éventuelle du radical Z'.

9. Procédé de préparation de dérivés de thiénopyridinone selon la Revendication 1, dans laquelle X représente un groupement hydroxy, amino, (alkyle inférieur)amino, alkoxy inférieur, (alkanoyle inférieur)oxy ou un groupement de formule :

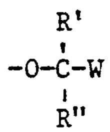 , 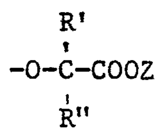 ou -NHCOOZ'

dans lequel R', R" et W sont tels que définis dans la Revendication 1 et Z' représente un radical alkyle inférieur, caractérisé en ce que l'on traite, au reflux, en présence d'un agent basique, un aldéhyde de formule générale :

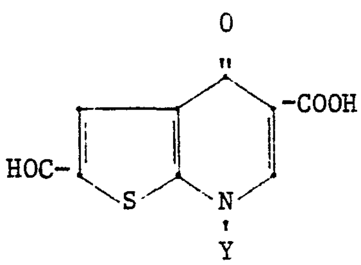

dans laquelle Y est tel que défini dans la Revendication 1, avec l'hydroxylamine, l'hydrazine, une (alkyle inférieure)hydrazine, une (alkyle inférieure)oxyamine, une (alkanoyle inférieure)oxyamine ou un composé de formule générale :

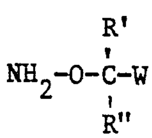 , 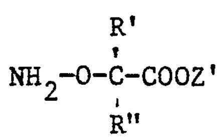 ou $NH_2$-NH-COOZ'

dans laquelle R', R", W et Z' sont tels que définis précédemment puis on acidifie avec un acide minéral et l'on transforme, si on le désire, le composé obtenu en ses sels pharmaceutiquement acceptables.

10. Composés de formule générale :

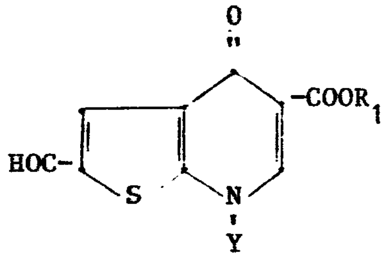

V

dans laquelle Y et $R_1$, qui peuvent être identiques ou différents, représentent chacun l'hydrogène ou un radical alkyle inférieur.

11. Composés selon la Revendication 10 dans laquelle $R_1$ est l'hydrogène.

12. Composés selon la Revendication 10 dans laquelle $R_1$ est tertiobutyle.

13. Procédé pour la préparation des composés selon la Revendication 10 caractérisé en ce que l'on traite un dérivé du thiophène de formule générale :

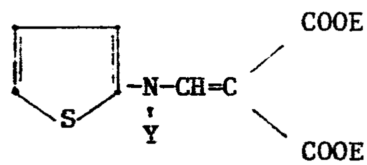

dans laquelle Y représente l'hydrogène ou un radical alkyle inférieur et E représente un radical alkyle inférieur avec l'oxychlorure de phosphore et le N-méthylformanilide, on soumet ensuite l'aldéhyde ainsi obtenu de formule générale :

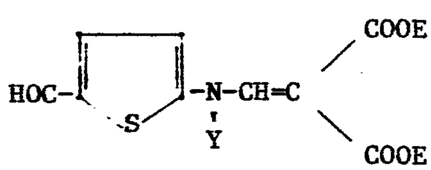

dans laquelle Y et E sont tels que définis ci-dessus, à une cyclisation thermique en présence d'acide polyphosphorique et, si nécessaire, on hydrolyse le produit ainsi obtenu de formule générale :

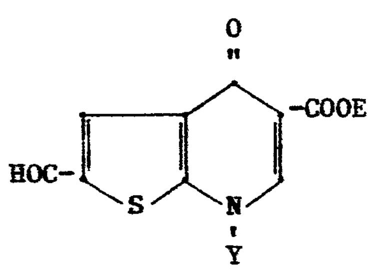

dans laquelle Y et E sont tels que définis ci-dessus, puis on isole l'acide libre de formule V à la Revendication 8, dans laquelle $R_1$ représente l'hydrogène.

14. A titre de produits intermédiaires utiles dans le procédé selon la Revendication 13, les composés de formule générale :

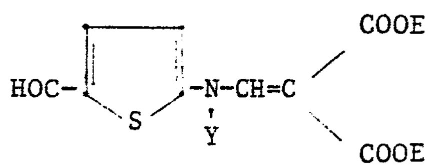

dans laquelle Y et E sont tels que définis dans la Revendication 13.

15. Composition pharmaceutique pour le traitement des infections bactériennes chez les mammifères contenant, comme ingrédient actif, un dérivé de thiénopyridinone selon la Revendication 1.

16. Composition selon la Revendication 15, sous forme d'unité de dosage.

17. Composition selon l'une des Revendications 15ou 16 contenant de 100 à 5000mg d'ingrédient actif par unité de dosage, en mélange avec un véhicule ou excipient pharmaceutique.